# EUROPEAN PATENT APPLICATION

(11) **EP 4 234 665 A1**
(43) Date of publication of application: **30.08.2023**
(21) Application number: 22158855.1
(22) Date of filing: 25.02.2022
(51) Int. Cl.: C11D 1/72, C07C 309/62, C11C 3/00, C11D 1/12, C11D 1/28, C11D 1/29, A61K 8/34, A61K 8/37, A61K 8/39, A61K 8/46, A61K 8/60

(54) **MACAÚBA OIL FOR THE PRODUCTION OF OLEOCHEMICALS**

(71) Applicant: BASF SE, 67056 Ludwigshafen am Rhein (DE)
(72) Inventor: SANCHEZ VALDIVIA, Agustin, 40789 Monheim (DE); HUESKEN, Hendrik, 40789 Monheim (DE); PICOLI, Allan Gustavo, 04794-000 sao Paulo (BR); SUDATI, Donato Giovanbatti, 22070 Veniano (IT)
(74) Representative: Maiwald GmbH

(57) **Abstract**

The present invention relates to a process of manufacturing a fatty alcohol composition comprising the step of converting oil extracted from a plant having an oil yield in tons per hectare per year of at least 6 t/ha/yr into the fatty alcohol composition. Further, the present invention relates to fatty alcohol composition obtained from the fruits of a plant having an oil yield in tons per hectare per year of at least 6 t/ha/yr and the use thereof in suitable applications.

## Description

The present invention relates to a process of manufacturing a fatty alcohol composition comprising the step of converting oil extracted from a plant having an oil yield in tons per hectare per year of at least 6 t/ha/yr into the fatty alcohol composition. Further, the present invention relates to fatty alcohol composition obtained from the fruits of a plant having an oil yield in tons per hectare per year of at least 6 t/ha/yr and the use thereof in suitable applications.

Numerous oil-based products are derived from renewable materials such as oil palm (principal source of palm oil). While such an approach is advantage since it safes the petroleum deposit it also provides several downsides. One issue is the deforestation in order to plant e.g. oil palm plantations, which aggravates the current climate change. Deforestation further leads to undesired loss of biodiversity and the loss of habitats for local tribes. In addition, particularly oil palms need tropical conditions and preferred temperatures between about 24 to 28 °C, monthly rainfalls of at least 100 mm/m², and a humidity between about 50 to 70%. These factors limit the possibility of a profitable cultivation.

At the same time the demand for renewable oil increases every year since the worldwide consume is increasing. Products derived from renewable oil can be found in every important industrial section, e.g. food products, pharmaceuticals, consumer goods, or energy (biodiesel).

Against this background, there is an ongoing need for a more environmental friendly alternative to known products derived from renewable oil such as palm oil. In particular, it was an object of the present invention to provide a fatty alcohol composition having an improved sustainability profile, as well as a process of manufacturing thereof. Further, it was an object of the present invention to provide a fatty alcohol composition, wherein the starting material is derived from plants that are less vulnerable against temperature fluctuation, as well as a process of manufacturing thereof. Further, it was an object to provide a surfactant having an improved sustainability profile, as well as a process of manufacturing thereof. Finally, it was an objection to provide a personal care composition, a cleaning composition, a nutrition formulation, a pharmaceutical formulation, or a crop formulation having an improved sustainability profile, as well as a process of manufacturing thereof. In this connection, a more environmental friendly alternative preferably provides at least one, more preferably at least two, still more preferably at least three, and in particular at least four, of the following impacts: reduced water demand, reduction of the loss of biodiversity, reduction of loss of habitats for local tribes, reduction of deforestation, improved recovery of degraded areas and springs and watersheds, improved retention of moisture in the soil.

It has surprisingly been found that at least one of these objects can be achieved by applying an oil extracted from a plant having an oil yield in tons per hectare per year of at least 6 t/ha/yr.

Thus, according to one aspect, the present invention relates to a process of manufacturing a fatty alcohol composition, the process comprising the step of
a) converting oil extracted from a plant having an oil yield in tons per hectare per year of at least 6 t/ha/yr into the fatty alcohol composition.

In the following, preferred embodiments of the above process are described in further detail. It is to be understood that each preferred embodiment is relevant on its own as well as in combination with other preferred embodiments.

In a preferred embodiment A1 of the first aspect, the plant is a palm, preferably a palm of the genus *Acrocomia,* more preferably a Macaúba palm, and in particular *Acrocomia aculeata* and/or
wherein the plant is a palm and the oil is extracted from the palm pulp and/or the palm kernel, preferably wherein the plant is Macaúba palm and the oil is extracted from the Macaúba kernel, and in particular wherein the plant is *Acrocomia aculeata* and the oil is extracted from *Acrocomia aculeata* kernel.

In a preferred embodiment A2 of the first aspect, in step a) the conversion is conducted under chemical or enzymatic conditions, preferably under chemical conditions.

In a preferred embodiment A3 of the first aspect, step a) involves
i) a hydrogenation, preferably a transesterification and a hydrogenation, in particular a transesterification followed by a hydrogenation or
ii) a hydrolysis, preferably a hydrolysis and a hydrogenation, in particular a hydrolysis followed by a hydrogenation.

In a preferred embodiment A4 of the first aspect, the fatty alcohol composition comprises at least 45 wt.-%, based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols.

In a preferred embodiment A5 of the first aspect, the plant has an oil yield in tons per hectare per year in the range of 6 to 30 t/ha/yr, preferably 7 to 20 t/ha/yr, more preferably of 8 to 15 t/ha/yr.

In a preferred embodiment A6 of the first aspect, the process further comprises
b) conversion of the fatty alcohols into derivatives thereof selected from the group consisting of fatty alcohol sulfates, fatty alcohol ethersulfates, fatty esters, fatty amines, fatty amides, fatty alcohol glycosides, fatty alkyl polyglucoside, fatty ethers, and fatty alcohol ethoxylate.

In a second aspect, the present invention relates to a fatty alcohol composition obtained by a process according to the first aspect.

In a preferred embodiment B1 of the second aspect, the fatty alcohol composition comprises at least 45 wt.-% based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols or C12-C14 fatty alcohols.

In a third aspect, the present invention relates to a fatty alcohol composition obtained from the fruits of a plant having an oil yield in tons per hectare per year of at least 6 t/ha/yr, wherein the oil obtained from the plant is converted into the fatty alcohol composition.

In a preferred embodiment C1 of the third aspect, the plant is a palm, preferably a palm of the genus *Acrocomia,* more preferably a Macaúba palm, and in particular *Acrocomia aculeata* and/or wherein the oil is obtained by extraction of the fruits, preferably wherein the plant is a palm and the oil is extracted from the palm pulp and/or the palm kernel, more preferably wherein the plant is Macaúba palm and the oil is extracted from the Macaúba kernel, and in particular wherein the plant is *Acrocomia aculeata* and the oil is extracted from *Acrocomia aculeata* kernel.

In a preferred embodiment C1 of the second aspect, the fatty alcohol composition comprises at least 45 wt.-% based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols or C12-C14 fatty alcohols.

In a fourth aspect, the present invention relates to the use of a fatty alcohol composition according to any one of claims 8 to 11 for manufacturing a C4 fatty alcohol, a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, a C22 fatty alcohol, or blends thereof.

In a fifth aspect, the present invention relates to a process of manufacturing a surfactant comprising the steps of
A) separating fatty alcohols selected from the group consisting of a C6 fatty alcohol, a C8 fatty alcohol, a C10 fatty alcohol, a C12 fatty alcohol, a C14 fatty alcohol, a C16 fatty alcohol, a C18 fatty alcohol, and a C20 fatty alcohol, from the fatty alcohol composition according to any one of claims 8 to 11,
B) optionally blending at least two of the separated fatty alcohols,
C) subsequently converting at least one of the separated fatty alcohols selected from the group consisting of a C6 fatty alcohol, a C8 fatty alcohol, a C10 fatty alcohol, a C12 fatty alcohol, a C14 fatty alcohol, a C16 fatty alcohol, a C18 fatty alcohol, and a C20 fatty alcohol into the respective fatty alcohol derivative selected from the group consisting of fatty alcohol sulfate, fatty alcohol ethersulfate, fatty ester, fatty amine, fatty alcohol glycoside, fatty alkyl polyglucoside, fatty ether, and fatty alcohol ethoxylate.

In a preferred embodiment E1 of the firth aspect, the fatty alcohol composition according to any one of claims 8 to 11 applied in step A) is first blended with a fatty alcohol composition obtained from a plant having an oil yield in tons per hectare per year of less than 6 t/ha/yr, preferably wherein the fatty alcohol composition obtained from a plant having an oil yield in tons per hectare per year of less than 6 t/ha/yr is derived from coconut oil (CNO), palm oil (PO), and/or palm kernel oil (PKO) or
wherein the fatty alcohol derivative obtained in step C) is blended with fatty alcohol derivatives obtained from a fatty alcohol composition obtained from a plant having an oil yield in tons per hectare per year of less than 6 t/ha/yr and a subsequent conversion into the respective fatty alcohol derivatives, preferably wherein the fatty alcohol composition obtained from a plant having an oil yield in tons per hectare per year of less than 6 t/ha/yr is derived from coconut oil (CNO), palm oil (PO), and/or palm kernel oil (PKO).

In a sixth aspect, the present invention relates to a surfactant obtained by the process according to the fifth aspect.

In a seventh aspect, the present invention relates to a process of manufacturing a C4 fatty alcohol, a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, a C22 fatty alcohol, and/or blends thereof, preferably a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, and/or blends thereof, more preferably a C8 fatty alcohol, a C10 fatty alcohol, a C12 fatty alcohol, a C14 fatty alcohol, a C16 fatty alcohol, a C18 fatty alcohol, and/or blends thereof, even more preferably a C10 fatty alcohol, a C12 fatty alcohol, a C14 fatty alcohol, a C16 fatty alcohol, and/or blends thereof, and in particular a C12 fatty alcohol, a C14 fatty alcohol, and/or blends thereof, comprising separating the C4 fatty alcohol, C6 fatty alcohol, C8 fatty alcohol, C10 fatty alcohol, C12 fatty alcohol, C14 fatty alcohol, C16 fatty alcohol, C18 fatty alcohol, C20 fatty alcohol, and/or C22 fatty alcohol, preferably the C6 fatty alcohol, C8 fatty alcohol, C10 fatty alcohol, C12 fatty alcohol, C14 fatty alcohol, C16 fatty alcohol, C18 fatty alcohol, and/or C20 fatty alcohol, more preferably the C8 fatty alcohol, C10 fatty alcohol, C12 fatty alcohol, C14 fatty alcohol, C16 fatty alcohol, and/or C18 fatty alcohol, even more preferably the C10 fatty alcohol, C12 fatty alcohol, C14 fatty alcohol, and/or C16 fatty alcohol, and in particular the C12 fatty alcohol and/or C14 fatty alcohol, from the fatty alcohol composition according to any one of claims 8 to 11 and optionally blending at least two of the separated fatty alcohols.

In an eighth aspect, the present invention relates to the use of the surfactant according to the sixth aspect in a personal care composition, a cleaning composition, a nutrition formulation, a pharmaceutical formulation, or a crop formulation.

In a ninth aspect, the present invention relates to a personal care composition, a cleaning composition, a nutrition formulation, a pharmaceutical formulation, or a crop formulation comprising a surfactant according to the sixth aspect.

### Detailed Description

Before describing in detail exemplary embodiments of the present invention, definitions which are important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±10 %, preferably ±8 %, more preferably ±5 %, even more preferably ±2 %. It is to be understood that the term "comprising" and "encompassing" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below. It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As used herein the term "does not comprise", "does not contain", or "free of" means in the context that the composition of the present invention is free of a specific compound or group of compounds, which may be combined under a collective term, that the composition does not comprise said compound or group of compounds in an amount of more than 0.8 % by weight, based on the total weight of the composition. Furthermore, it is preferred that the composition according to the present invention does not comprise said compounds or group of compounds in an amount of more than 0.5 % by weight, preferably the composition does not comprise said compounds or group of compounds at all.

When referring to compositions and the weight percent of the therein comprised ingredients it is to be understood that according to the present invention the overall amount of ingredients does not exceed 100% (± 1% due to rounding).

The term "personal care composition" refers to any topical and oral product that can be used at least once daily by the costumer as an everyday care product for the human body, e.g. for face, hair, body, or oral care. The personal care composition may comprise one or more active agents, e.g., organic and/or inorganic UV filters, as well as other ingredients or additives, e.g., emulsifiers, emollients, viscosity regulators, stabilizers, preservatives, or fragrances. Suitable daily care composition are according to the present invention, e.g. leave-on face and body care products and rinse-off face and body care products.

Suitable leave-on face and body care products are, e.g. sunscreen compositions, decorative preparations, and skin care preparations.

The term "sunscreen composition" or "sunscreen" refers to any topical product, which absorbs and which may further reflect and scatter certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, foams, powders, or gels. According to the present invention the sunscreen composition may comprise one or more active agents, e.g., organic and inorganic UV filters, as well as other ingredients or additives, e.g., emulsifiers, emollients, viscosity regulators, stabilizers, preservatives, or fragrances.

Suitable decorative preparations are, e.g., lipsticks, nail varnishes, eye shadows, mascaras, dry and moist make-up, rouge, powders, depilatory agents and suntan lotions.

Suitable skin care preparations are e.g., moisturizing, refining, and lifting preparations. The cited daily care compositions can be in the form of creams, ointments, pastes, foams, gels, lotions, powders, make-ups, sprays, sticks or aerosols.

The term "UV filter" or "ultraviolet filter" as used herein refers to organic or inorganic compounds, which can absorb and may further reflect and scatter UV radiation caused by sunlight. UV-filter can be classified based on their UV protection curve as UV-A, UV-B, or broadband filters.

In general, UV light can be divided into UV-A radiation (320 - 400 nm) and UV-B radiation (290 - 320 nm). The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. The term "critical wavelength" is defined as the wavelength at which the area under the UV protection curve (% protection versus wavelength) represents 90 % of the total area under the curve in the UV region (290-400 nm). For example, a critical wavelength of 370 nm indicates that the protection of the sunscreen composition is not limited to the wavelengths of UV-B, i.e. wavelengths from 290-320 nm, but extends to 370 nm in such a way that 90 % of the total area under the protective curve in the UV region are reached at 370 nm.

Suitable rinse-off face and body care products are, e.g. shampoo, conditioner, shower gel, body scrub, face scrub, and hand soap.

The term "emollient" relates to cosmetic specific oils used for protecting, moisturizing and lubricating the skin. The word emollient is derived from the Latin word *mollire*, to soften. In general, emollients prevent evaporation of water from the skin by forming an occlusive coating. They can be divided into different groups depending on their polarity index.

The term "polarity index" refers to non-polar or polar oils. Non-polar oils are mainly based on hydrocarbons and lack an electronegative element, such as oxygen. In contrast, polar oils contain heteroatoms that differ in electronegativity, which results in a dipole moment. However, such oils are still insoluble in water, i.e. hydrophobic. The polarity index can be determined by measuring the interfacial tension between the respective oil and water.

The term "administration" refers to the application of a sunscreen or daily care composition to the skin of a person.

The term "fatty alcohol" as used herein is directed to linear or branched, preferably linear, primary alcohols. Fatty alcohols may comprise from 4 to 26 carbon atoms. According to the present invention, the term fatty alcohol encompasses saturated and unsaturated alcohol. The double bond of an unsaturated fatty alcohol can give either cis or trans isomers. According to the present invention, the term fatty alcohol encompasses saturated and unsaturated alcohols. 1-Butanol, 1-hexanol, 1-octanol, 1-decanol, lauryl alcohol, myristyl alcohol, cetyl alcohol, palmitoyl alcohol, stearyl alcohol, oleyl alcohol, arachidyl alcohol, behenyl alcohol, erucyl alcohol, lignoceryl alcohol, and ceryl alcohol should be named in this connection.

The term "fatty acid" as used herein is directed to linear or branched, preferably linear, primary carboxylic acids. Fatty acids may comprise from 4 to 26 carbon atoms. According to the present invention, the term fatty acid encompasses saturated and unsaturated acids. The double bond of an unsaturated fatty acid can give either cis or trans isomers. Caprylic acid, capric acid, lauric acid, myristic acid, myristoleic acid, palmitic acid, palmitoleic acid, sapienic acid, stearic acid, oleic acid, elaidic acid, vaccenic acid, linoleic acid, linoelaidic acid, α-Linolenic acid, arachidic acid, arachidonic acid, eicosapentaenoic acid, erucic acid, behenic acid, docosahexaenoic acid, lignoceric acid, and cerotic acid should be named in this connection.

The term "fatty acid-based surfactant" as used herein denotes a surfactant that originates from a reaction of the primary carboxylic group of a fatty acid.

The prefix Cₙ-Cₘ indicates in each case the possible number of carbon atoms in the group. The term "alkyl" as used herein denotes in each case a linear or branched alkyl group having usually from 1 to 30 carbon atoms, preferably 4 to 26 or of 1 to 6 or of 1 to 3 carbon atoms. Examples of an alkyl group are methyl, ethyl, n-propyl, iso-propyl, n-butyl, 2-butyl, iso-butyl, tert-butyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The term "alkoxy" as used herein denotes in each case a linear or branched alkyl group which is bonded via an oxygen atom and has usually from 1 to 6 carbon atoms, preferably 1 to 2 carbon atoms, more preferably 1 carbon atom. Examples of an alkoxy group are methoxy, ethoxy, n-propoxy, iso-propoxy, n-butyloxy, 2-butyloxy, iso-butyloxy, tert.-butyloxy, and the like.

As used herein, the term "alkylene" refers to a linking linear or branched alkylene group having usually from 1 to 4 carbon atoms, e.g. 1, 2, 3, or 4 carbon atoms. The alkylene group bridges a certain group to the remainder of the molecule. Preferred alkylene groups include methylene (CH₂), ethylene (CH₂CH₂), propylene (CH₂CH₂CH₂) and the like. A skilled person understands that, if it is referred, e.g., to CH₂ that the carbon atom being tetravalent has two valences left for forming a bridge (-CH₂-). Similarly, when it is referred, e.g., to CH₂CH₂, each carbon atom has one valence left for forming a bridge (-CH₂CH₂-). Furthermore, when it is referred, e.g., to CH₂CH₂CH₂, each terminal carbon atom has one valence left for forming a bridge (-CH₂CH₂CH₂-).

The term "heterocyclic" or "heterocyclyl" includes, unless otherwise indicated, in general a 3-to 9-membered, preferably a 4- to 8-membered or 5- to 7-membered, more preferably 5- or 6-membered, in particular 6-membered monocyclic ring. The heterocycle may be saturated, partially or fully unsaturated, or aromatic, wherein saturated means that only single bonds are present, and partially or fully unsaturated means that one or more double bonds may be present in suitable positions, while the Hückel rule for aromaticity is not fulfilled, whereas aromatic means that the Hückel (4n + 2) rule is fulfilled. The heterocycle typically comprises one or more, e.g. 1, 2, 3, or 4, preferably 1, 2, or 3 heteroatoms selected from N, O and S as ring members, where S-atoms as ring members may be present as S, SO or SO₂. The remaining ring members are carbon atoms. In a preferred embodiment, the heterocycle is an aromatic heterocycle, preferably a 5- or 6-membered aromatic heterocycle comprising one or more, e.g. 1, 2, 3, or 4, preferably 1, 2, or 3 heteroatoms selected from N, O and S as ring members, where S-atoms as ring members may be present as S, SO or SO₂. Examples of aromatic heterocycles are provided below in connection with the definition of "hetaryl". "Hetaryls" or "heteroaryls" are covered by the term "heterocycles". The saturated or partially or fully unsaturated heterocycles usually comprise 1, 2, 3, 4 or 5, preferably 1, 2 or 3 heteroatoms selected from N, O and S as ring members, where S-atoms as ring members may be present as S, SO or SO₂. The skilled person is aware that S, SO or SO₂ is to be understood as follows:

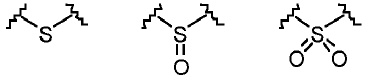

Further, a skilled person is aware that resonance structures of the oxidized forms may be possible. Saturated heterocycles include, unless otherwise indicated, in general 3- to 9-membered, preferably 4- to 8-membered or 5- to 7-membered, more preferably 5- or 6-membered monocyclic rings comprising 3 to 9, preferably 4 to 8 or 5 to 7, more preferably 5 or 6 atoms comprising at least one heteroatom, such as pyrrolidine, tetrahydrothiophene, tetrahydrofuran, piperidine, tetrahydropyran, dioxane, morpholine or piperazine.

The term "aryl" or "aromatic carbocycle" preferably includes 6-membered aromatic carbocyclic rings based on carbon atoms as ring members. A preferred example is phenyl.

The term "oil palm" as used herein denotes a species of palm, which is also known as "*Elaeis guineensis*". It is the principal source of "palm oil".

The term "coconut tree" as used herein denotes a member of the palm tree family (*Arecaceae*) and is also referred to as *Cocos nucifera.* It is the principal source for "coconut oil".

The term "Macaúba palm" as used herein denotes a species of palm. Exemplary species are known as "*Acrocomia aculeate*" (also known as "macaíba", "boicaiuva", "macaúva", "coco-de-catarro", "coco-baboso", and "coco-de-espinho"), "*Acrocomia hassleri*", and "*Acrocomia totei*". Macaúba palms can grow high, e.g. up to about 15 m. The Macaúba fruit comprises pulp and kernel.

The term "pulp" as used herein refers to inner flesh of a fruit.

The term "kernel" as used herein is interchangeable with "seed" or "almond".

The term "cleaning composition" as used herein encompasses home care formulation, industrial care formulation, and institutional care formulation. Home care formulations are typically used by private costumers, whereas industrial care formulations are typically used by the industry, and institutional care formulations are typically used in e.g. clinics and nursing homes. It is however also possible that the respective formulations can be used in different areas than intended. Hence, the institutional care formulation may also be used by private costumers or the industry and vic verca. Typically cleaning compositions are e.g. for the laundry, dishwashing, hard surface cleaning, food service and kitchen hygiene, food and beverage processing, commercial laundry, sanitation, institutional cleaning, industrial cleaning, and vehicle and transportation care.

The term "nutrition formulation" as used herein encompasses food and feed formulations. The nutrition formulation can have any suitable form, e.g. liquid or solid and can be administered or uptaken in any suitable manner, e.g. orally, parenterally, or rectally.

The term "pharmaceutical formulation" as used herein refers to any suitable pharmaceutical formulation, which may e.g. be administered in any suitable manner such as by oral, transdermal, parenteral, nasal, vaginal, or rectal application. Suitable solid pharmaceutical formulation can be in form of tablets, suppositories, or capsules or in form of a spray. Suitable transdermal pharmaceutical formulations encompass patches or formulations such as sprays, lotions, creams, oils, foams, ointments, powders, or gels. Liquid pharmaceutical formulations are preferably administered orally, parenterally, or nasal.

The term "liquid" as used herein also encompasses semi-solid conditions, wherein the fluid has an increased viscosity (e.g. creamy, gels, ointments).

The term "crop formulation" as used herein encompasses pesticide formulations, fungicide formulations, and herbicide formulations.

The term "oil yield in tons per hectare per year" as used herein is directed to the oil derived from the fruit of the plant via e.g. extraction, wherein the fruit comprises the pulp and the kernel. It refers to the oil produced per hectare. It is to be understood that the value refers to the oil yield obtained from a monoculture, wherein the plants are cultivated under standard conditions, which depend on the respective plant and are known to the skilled person. Hence, in the event that the plant is not cultivated in a monoculture (e.g. on a cattle field), the respective value for this particular cultivation may be reduced. Typically, oil palm has an oil yield in tons per hectare per year of about 3.8 t/ha/yr, rapeseed has an oil yield in tons per hectare per year of about 0.8 t/ha/yr, sunflower has an oil yield in tons per hectare per year of about 0.7 t/ha/yr, and soya has an oil yield in tons per hectare per year of about 0.6 t/ha/yr.

The term "monoculture" as used herein denotes the practice of growing one plant, e.g. Macaúba palm, in a field at a time. On the example of Macaúba palm, about 500 to about 600 palms can be planted per hectare. In this connection, it is preferred that the minimum distance between the tress is about 3.5 to 4.5 meters. This number varies depending on e.g. the soil.

The term "agroforestry" as used herein denotes a land use management system in which trees or shrubs are grown around or among other plant such as other trees or other shrubs or crops or pastureland. It is to be understood that not only one further plant can be present in agroforestry. On the example of Macaúba palm, e.g. about 250 to about 360 or about 325 to about 350, trees can be planted per hectare. In this connection, suitable crops that may be planted together with Macaúba palm are exemplarily beans, mandioca, corn, cereals, sunflower, peanut, rapeseed, soya, and mixtures thereof.

The term "silvopastoral" as used herein denotes a land use management system in which trees and optionally forage are planted within the grazing of domesticated animals. On the example of Macaúba palm, e.g. about 275 to about 450 or about 375 to about 400, trees can be planted per hectare.

Preferred embodiments regarding the process of manufacturing a fatty alcohol composition or a surfactant or a specific fatty alcohol, the fatty alcohol compositions as well as the use thereof, the use of the surfactant and the products comprising the same are described hereinafter. It is to be understood that the preferred embodiments of the invention are preferred alone or in combination with each other.

As indicated above, the present invention relates in one embodiment to a process of manufacturing a fatty alcohol composition, the process comprising the step of
a) converting oil extracted from a plant having an oil yield in tons per hectare per year of at least 6 t/ha/yr into the fatty alcohol composition.

In a preferred embodiment, the plant is a palm, preferably a palm of the genus *Acrocomia,* more preferably a Macaúba palm, still more preferably *Acrocomia hassleri*, *Acrocomia totei,* and/or *Acrocomia aculeata*, and in particular *Acrocomia aculeata.*

In a preferred embodiment, the plant is a palm and the oil is extracted from the palm pulp and/or the palm kernel.

In a preferred embodiment, the plant is Macaúba palm and the oil is extracted from the Macaúba kernel, preferably wherein the plant is *Acrocomia hassleri*, *Acrocomia totei,* and/or *Acrocomia aculeata* and the oil is extracted from more preferably *Acrocomia hassleri* kernel, *Acrocomia totei* kernel, and/or *Acrocomia aculeata* kernel, and in particular wherein the plant is *Acrocomia aculeata* and the oil is extracted from *Acrocomia aculeata* kernel.

In another preferred embodiment, plant is Macaúba palm and the oil is extracted from the Macaúba pulp, and in particular wherein the plant is *Acrocomia aculeata* and the oil is extracted from *Acrocomia aculeata* pulp.

In another preferred embodiment, plant is Macaúba palm and the oil is extracted from the Macaúba pulp and kernel, and in particular wherein the plant is *Acrocomia aculeata* and the oil is extracted from *Acrocomia aculeata* pulp and kernel.

In a preferred embodiment, the plant can sufficiently grow under tropical and subtropical conditions.

In a preferred embodiment, the plant can sufficiently grow in regions from the 30^{th} parallel north to the 28^{th} parallel south, preferably from the 25^{th} parallel north to the 25^{th} parallel south.

In a preferred embodiment, the plant sufficiently grows at a temperature range of 18 to 30 °C, more preferably of 20 to 28 °C. In this connection it is to be understood that the temperature range is the average temperature over one year. Hence, the plant is preferably less vulnerable to temperature fluctuation.

The term "sufficiently grow" as used herein denotes that the claimed oil yield is achievable under standard cultivation.

In addition, particularly oil palm need tropical conditions and preferred temperatures between about 24 to 28 °C, monthly rainfalls of at least 100 mm/m², and a humidity between about 50 to 70%. These factors limit the possibility of a profitable cultivation.

In a preferred embodiment, the process provides a reduced water demand.

In a preferred embodiment, the process provides a reduction of the loss of biodiversity.

In a preferred embodiment, the process provides a reduction of loss of habitats for local tribes.

In a preferred embodiment, the process provides a reduction of deforestation.

In a preferred embodiment, the process provides an improved recovery of degraded areas and/or springs and watersheds.

In a preferred embodiment, the process provides an improved retention of moisture in the soil. In this connection it is to be understood that the above-outlined reductions or improvements are compared to plants having an oil yield in tons per hectare per year of less than 6 t/ha/yr, preferably compared to oil palm.

In a preferred embodiment, the oil extracted from a plant having an oil yield in tons per hectare per year of at least 6 t/ha/yr is the crude oil, i.e. not further treated after the extraction from the plant.

In another preferred embodiment, the oil extracted from a plant having an oil yield in tons per hectare per year of at least 6 t/ha/yr is the filtered oil, i.e. wherein the crude oil is first filtered by any known in the art filtering systems and then used in the process. A suitable filtration process is e.g. press filtration.

In a preferred embodiment, in step a) the conversion is conducted under chemical or enzymatic conditions, preferably under chemical conditions.

In a preferred embodiment, step a) involves
i) a hydrogenation, preferably a transesterification and a hydrogenation, in particular a transesterification followed by a hydrogenation or
ii) a hydrolysis, preferably a hydrolysis and a hydrogenation, in particular a hydrolysis followed by a hydrogenation.

Any suitable transesterification method can be conducted. Preferably, the transesterification is conducted in the presence of simple alcohols such as methanol, ethanol, propanol, and/or isopropanol providing glycerol and the respective esters. In a preferred embodiment, the transesterification is conducted in the presence of methanol. The reaction can further comprise the addition of a catalysts. Preferably, the alcohol is provided in excess.

Any suitable hydrogenation method can be conducted. Preferably, the hydrogenation is conducted in the presence of a solid catalyst and hydrogen. Preferably, the hydrogenation is performed at a pressure of about 10 to about 350 bar, more preferably of about 200 to about 300 bar. Preferably the hydrogenation is performed at a temperature of about 50 to about 300 °C, more preferably of about 100 to about 270 °C.

In a preferred embodiment, the hydrogenation is conducted partially, i.e. that the fatty alcohol composition still comprises unsaturated moieties.

In another preferred embodiment, complete hydrogenation is conducted, i.e. that the fatty alcohol composition does not comprise unsaturated moieties.

In a preferred embodiment, the fatty alcohol composition comprises at least 45 wt.-% based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols or C12-C14 fatty alcohols.

In a preferred embodiment, the fatty alcohol composition comprises at least 85 wt.-% based on the total weight of the fatty alcohol composition, of C4-C22 fatty alcohols, preferably C10-C22 fatty alcohols, more preferably C12-C20 fatty alcohols, even more preferably C12-C20 fatty alcohols, and in particular C12-C18 fatty alcohols.

In a preferred embodiment, the fatty alcohol composition comprises at least 10 wt.-% of C16 fatty alcohols and at least 75 wt.-% of C18 fatty alcohols, each based on the total weight of the fatty alcohol composition.

In a preferred embodiment, the fatty alcohol composition comprises 10 to 25 wt.-% of C16 fatty alcohols and 75 to 90 wt.-% of C18 fatty alcohols, each based on the total weight of the fatty alcohol composition.

In a preferred embodiment, the fatty alcohol composition comprises at least 80 wt.-%, preferably at least 90 wt.-%, and in particular at least 95 wt.-%, based on the total weight of the fatty alcohol composition, of C12-14 fatty alcohols.

In a preferred embodiment, the fatty alcohol composition comprises at least 80 wt.-%, preferably at least 90 wt.-%, and in particular at least 95 wt.-%, based on the total weight of the fatty alcohol composition, of C12-18 fatty alcohols.

In a preferred embodiment, the fatty alcohol composition comprises at least 2 wt.-% of C10 fatty alcohols, at least 35 wt.-% of C12 fatty alcohols, at least 5 wt.-% of C14 fatty alcohols, and at least 4 wt.-% of C16 fatty alcohols, each based on the total weight of the fatty alcohol composition.

In a preferred embodiment, the fatty alcohol composition comprises 3 to 7 wt.-% of C8 fatty alcohols, 2 to 6 wt.-% of C10 fatty alcohols, 35 to 45 wt.-% of C12 fatty alcohols, 5 to 13 wt.-% of C14 fatty alcohols, and 4 to 10 wt.-% of C16 fatty alcohols, each based on the total weight of the fatty alcohol composition.

In a preferred embodiment, the fatty alcohol composition comprises at least 90 wt.-% of C8 fatty alcohols, based on the total weight of the fatty alcohol composition.

In a preferred embodiment, the fatty alcohol composition comprises at least 90 wt.-% of C10 fatty alcohols, based on the total weight of the fatty alcohol composition.

In a preferred embodiment, the fatty alcohol composition comprises at least 90 wt.-% of C12 fatty alcohols, based on the total weight of the fatty alcohol composition.

In a preferred embodiment, the fatty alcohol composition comprises at least 90 wt.-% of C14 fatty alcohols, based on the total weight of the fatty alcohol composition.

In a preferred embodiment, the fatty alcohol composition comprises at least 90 wt.-% of C16 fatty alcohols, based on the total weight of the fatty alcohol composition.

In a preferred embodiment, the fatty alcohol composition comprises at least 90 wt.-% of C18 fatty alcohols, based on the total weight of the fatty alcohol composition.

In a preferred embodiment, the plant has an oil yield in tons per hectare per year in the range of at least 7 t/ha/yr, preferably at least 8 t/ha/yr.

In a preferred embodiment, the plant has an oil yield in tons per hectare per year in the range of 6 to 30 t/ha/yr, preferably 7 to 20 t/ha/yr, more preferably of 8 to 15 t/ha/yr or of 8 to 12 t/ha/yr or of 8 to 11 t/ha/yr.

In a preferred embodiment, the process further comprises
b) conversion of the fatty alcohols into derivatives thereof selected from the group consisting of fatty alcohol sulfates, fatty alcohol ethersulfates, fatty esters, fatty amines, fatty amides, fatty alcohol glycosides, fatty alkyl polyglucoside, fatty ethers, and fatty alcohol ethoxylate.

Suitable fatty alcohol sulfates can be express by the general formula (I)

R-O-SO3M (I),

wherein R is saturated or unsaturated C4-C24-alkyl, preferably C6-C20-alkyl, and in particular C12-C14-alkyl, and M is a suitable counter ion, such as triethanolammonium, Na+, K+ or NH4+, preferably sodium. Fatty alcohol sulfates are accessible via any suitable known in the art method.

Suitable fatty alcohol ethersulfates can be express by the general formula (II)

R-O(CH2CH2O)n-SO3M (II),

wherein R is saturated or unsaturated C4-C24-alkyl, preferably C6-C20-alkyl, and in particular C12-C14-alkyl, n is an average number of 1 to 30, preferably of 1 to 10, more preferably of 1 to 5, and M is a suitable counter ion, such as triethanolammonium, Na+, K+ or NH4+, preferably sodium. Fatty alcohol ethersulfates are accessible via any suitable known in the art method.

Suitable fatty esters can be expressed by the general formula (III)

R-O-CO-R1 (III),

wherein R is saturated or unsaturated C4-C24-alkyl, preferably C6-C20-alkyl, and in particular C12-C14-alkyl, and R1 is C1-C24-alkyl, C1-C4-alkoxy, aryl, benzyl, or heterocyclyl. Fatty esters are accessible via any suitable known in the art method.

The term "fatty amines" as used herein encompasses primary fatty amines, secondary amines, ternary amines, ethoxylated fatty amines, fatty amine oxides, and amine salts.

Suitable fatty amines (e.g. primary fatty amines, secondary amines, ternary amines) can be expressed by the general formula (IV)

R-NR2R2' (IV),

wherein R is saturated or unsaturated C4-C24-alkyl, preferably C6-C20-alkyl, and in particular C12-C14-alkyl, and R2 and R2' are independently H, C1-C4-alkyl, or C1-C8-alkylene-NR21R22, wherein R21 and R22 are independently C1-C4-alkyl.

Suitable ethoxylated fatty amines can be expressed by the general formula (V)

R-NR3R4 (V),

wherein R is saturated or unsaturated C4-C24-alkyl, preferably C6-C20-alkyl, and in particular C12-C14-alkyl, R3 and R4 are independently H or -(CH2CH2O)n-N, n is an average number of 1 to 20, preferably of 1.5 to 10, N is H or C1-C4-alkyl.

Suitable fatty amine oxides can be expressed by the general formula (VI)

R-NR5R6O (VI),

wherein R is saturated or unsaturated C4-C24-alkyl, preferably C6-C20-alkyl, and in particular C12-C14-alkyl, and R5 and R6 are independently C1-C4-alkyl.

Suitable amine salts are e.g. quaternary amines having the general formula (VII)

R-NR7R8R9Q (VII),

wherein R is saturated or unsaturated C4-C24-alkyl, preferably C6-C20-alkyl, and in particular C12-C14-alkyl, R7, R8, and R9 are independently H or C1-C16-alkyl, preferably H or C4-C10-alkyl, and Q is a halide, hydrogensulfate, bicarbonate, benzoate, formate, acetate, propionate and butyrate, preferably chloride. In this connection, it is to be understood that a positive charge is on the nitrogen and a negative charge is on Q.

In a preferred embodiment, the amine salt has the general formula (VII)

R-NR7R8R9Q (VII),

wherein R is saturated or unsaturated C12-C20-alkyl, preferably C12-C18-alkyl, and in particular C12-C14-alkyl, R7, R8, and R9 are independently H or C1-C5-alkyl, preferably methyl, and Q is a halide, preferably chloride.

Fatty amines are accessible via any suitable known in the art method.

Suitable fatty alcohol glycosides can be expressed by the general formula (VIII)

R-O-G (VIII),

wherein R is saturated or unsaturated C4-C24-alkyl, preferably C6-C20-alkyl, and in particular C12-C14-alkyl, and G is a glucose moiety, preferably connected via the C1 carbon atom. Fatty alcohol glycosides are accessible via any suitable known in the art method.

Suitable fatty alkyl polyglucoside can be expressed by the general formula (IX)

R-O-(G)g-H (IX),

wherein R is saturated or unsaturated C4-C24-alkyl, preferably C6-C20-alkyl, and in particular C8-C10-alkyl or C8-C14-alkyl or C12-C14-alkyl, G is a glucose moiety, preferably connected via the C1 and C4 carbon atom, and g is an average number of 1 to 50, preferably of 5 to 20. Fatty alcohol glycosides are accessible via any suitable known in the art method.

Suitable fatty ethers can be expressed by the general formula (X)

R-O-R10 (X),

wherein R is saturated or unsaturated C4-C24-alkyl, preferably C6-C20-alkyl, more preferably C8-C14-alkyl, and in particular C12-C14-alkyl, and R10 is C1-C10-alkyl, C1-C4-alkoxy, aryl, benzyl, or heterocyclyl. Fatty ethers are accessible via any suitable known in the art method.

Suitable fatty alcohol ethoxylate can be expressed by the general formula (XI)

R-O-(CH2CH2O)m-R11 (XI),

wherein R is saturated or unsaturated C4-C24-alkyl, preferably C6-C20-alkyl, more preferably C12-C18-alkyl, still more preferably C12-C16-alkyl, and in particular C12-C14-alkyl, m is an average number of 1 to 50, preferably of 1.5 to 10, and R11 is H, C1-C4-alkyl, C1-C4-alkoxy, aryl, benzyl, or heterocyclyl. Fatty alcohol ethoxylates are accessible via any suitable known in the art method.

Suitable fatty amides can be expressed by the general formula (XII)

R-N-CO-R12 (XII),

wherein R is saturated or unsaturated C4-C24-alkyl, preferably C6-C20-alkyl, and in particular C12-C14-alkyl, and R12 is C3-C24-alkyl, C1-C4-alkoxy, aryl, benzyl, heterocyclyl, or C1-C8-alkylene-NR13R13', wherein R13 and R13' are independently C1-C4-alkyl. Fatty amides are accessible via any suitable known in the art method, e.g. by first converting the fatty alcohol into the respective fatty amine, followed by a conversion into the respective fatty amide.

As indicated above, the present invention further relates to a fatty alcohol composition obtained by the above-outlined process according.

In a preferred embodiment, the fatty alcohol composition comprises
1 to 20 wt.-% of a C8 fatty alcohol,
1 to 8 wt.-% of a C10 fatty alcohol,
30 to 48 wt.-% of a C12 fatty alcohol,
5 to 15 wt.-% of a C14 fatty alcohol,
4 to 13 wt.-% of a C16 fatty alcohol,
15 to 42 wt.-% of a C18 fatty alcohol, and
0 to 5 wt.-% of a C20 fatty alcohol,
each based on the total weight of the fatty alcohol composition. Said fatty alcohol composition is preferably obtained from oil extracted from Macaúba kernel.

In a preferred embodiment, the fatty alcohol composition comprises
3 to 7 wt.-%, preferably 4 to 6 wt.-%, of a C8 fatty alcohol,
2 to 6 wt.-%, preferably 3 to 5 wt.-%, of a C10 fatty alcohol,
36 to 46 wt.-%, preferably 38 to 42 wt.-%, of a C12 fatty alcohol,
6 to 13 wt.-%, preferably 8 to 11 wt.-%, of a C14 fatty alcohol,
5 to 11 wt.-%, preferably 6 to 9 wt.-%, of a C16 fatty alcohol,
25 to 40 wt.-%, preferably 30 to 38 wt.-% of a C18 fatty alcohol, and
0 to 4 wt.-%, preferably 0 to 3 wt.-%, of a C20 fatty alcohol,
each based on the total weight of the fatty alcohol composition. Said fatty alcohol composition is preferably obtained from oil extracted from Macaúba kernel.

In a preferred embodiment, the fatty alcohol composition comprises
0 to 5 wt.-%, preferably 0 to 3 wt.-%, and in particular 0 to 2 wt.-%, of a C10 fatty alcohol,
0 to 6 wt.-%, preferably 0 to 5 wt.-%, and in particular 1 to 4 wt.-%, of a C12 fatty alcohol,
0 to 6 wt.-%, preferably 0 to 5 wt.-%, and in particular 1 to 4 wt.-%, of a C14 fatty alcohol,
10 to 35 wt.-%, preferably 13 to 32 wt.-%, and in particular 15 to 30 wt.-%, of a C16 fatty alcohol,
55 to 85 wt.-%, preferably 60 to 80 wt.-%, and in particular 65 to 75 wt.-%, of a C18 fatty alcohol,
0 to 4 wt.-%, preferably 0 to 3 wt.-%, and in particular 0 to 2 wt.-%, of a C20 fatty alcohol,
each based on the total weight of the fatty alcohol composition. Said fatty alcohol composition is preferably obtained from oil extracted from Macaúba pulp.

In a preferred embodiment, the fatty alcohol composition comprises at least 45 wt.-% based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols or C12-C14 fatty alcohols.

In a preferred embodiment, the fatty alcohol composition comprises at least 85 wt.-% based on the total weight of the fatty alcohol composition, of C4-C22 fatty alcohols, preferably C10-C22 fatty alcohols, more preferably C12-C20 fatty alcohols, even more preferably C12-C20 fatty alcohols, and in particular C12-C18 fatty alcohols.

In a preferred embodiment, the fatty alcohol composition comprises at least 10 wt.-% of C16 fatty alcohols and at least 75 wt.-% of C18 fatty alcohols, each based on the total weight of the fatty alcohol composition.

In a preferred embodiment, the fatty alcohol composition comprises 10 to 25 wt.-% of C16 fatty alcohols and 75 to 90 wt.-% of C18 fatty alcohols, each based on the total weight of the fatty alcohol composition.

In a preferred embodiment, the fatty alcohol composition comprises at least 80 wt.-%, preferably at least 90 wt.-%, and in particular at least 95 wt.-%, based on the total weight of the fatty alcohol composition, of C12-14 fatty alcohols.

In a preferred embodiment, the fatty alcohol composition comprises at least 80 wt.-%, preferably at least 90 wt.-%, and in particular at least 95 wt.-%, based on the total weight of the fatty alcohol composition, of C12-18 fatty alcohols.

In a preferred embodiment, the fatty alcohol composition comprises at least 2 wt.-% of C10 fatty alcohols, at least 35 wt.-% of C12 fatty alcohols, at least 5 wt.-% of C14 fatty alcohols, and at least 4 wt.-% of C16 fatty alcohols, each based on the total weight of the fatty alcohol composition.

In a preferred embodiment, the fatty alcohol composition comprises 3 to 7 wt.-% of C8 fatty alcohols, 2 to 6 wt.-% of C10 fatty alcohols, 35 to 45 wt.-% of C12 fatty alcohols, 5 to 13 wt.-% of C14 fatty alcohols, and 4 to 10 wt.-% of C16 fatty alcohols, each based on the total weight of the fatty alcohol composition.

In a preferred embodiment, the fatty alcohol composition comprises at least 90 wt.-% of C8 fatty alcohols, based on the total weight of the fatty alcohol composition.

In a preferred embodiment, the fatty alcohol composition comprises at least 90 wt.-% of C10 fatty alcohols, based on the total weight of the fatty alcohol composition.

In a preferred embodiment, the fatty alcohol composition comprises at least 90 wt.-% of C12 fatty alcohols, based on the total weight of the fatty alcohol composition.

In a preferred embodiment, the fatty alcohol composition comprises at least 90 wt.-% of C14 fatty alcohols, based on the total weight of the fatty alcohol composition.

In a preferred embodiment, the fatty alcohol composition comprises at least 90 wt.-% of C16 fatty alcohols, based on the total weight of the fatty alcohol composition.

In a preferred embodiment, the fatty alcohol composition comprises at least 90 wt.-% of C18 fatty alcohols, based on the total weight of the fatty alcohol composition.

As indicated above, the present invention further relates to a fatty alcohol composition obtained from the fruits of a plant having an oil yield in tons per hectare per year of at least 6 t/ha/yr, wherein the oil obtained from the plant is converted into the fatty alcohol composition.

Preferred embodiments are already above-outlined in the process of manufacturing a fatty alcohol composition and shall apply for the fatty alcohol composition, as well. Particular preferred embodiment are mentioned in the following.

In a preferred embodiment, the plant is a palm, preferably a palm of the genus *Acrocomia,* more preferably a Macaúba palm, still more preferably *Acrocomia hassleri*, *Acrocomia totei,* and/or *Acrocomia aculeata,* and in particular *Acrocomia aculeata* and/or wherein the oil is obtained by extraction of the fruits, preferably wherein the plant is a palm and the oil is extracted from the palm pulp and/or the palm kernel, more preferably wherein the plant is Macaúba palm and the oil is extracted from the Macaúba kernel, still more preferably wherein the plant is *Acrocomia hassleri*, *Acrocomia totei,* and/or *Acrocomia aculeata* and the oil is extracted from more preferably *Acrocomia hassleri* kernel, *Acrocomia totei* kernel, and/or *Acrocomia aculeata* kernel, and in particular wherein the plant is *Acrocomia aculeata* and the oil is extracted from and/or *Acrocomia aculeata* kernel.

In a preferred embodiment, the fatty alcohol composition comprises
1 to 20 of wt.-% of a C8 fatty alcohol,
1 to 8 of wt.-% of a C10 fatty alcohol,
30 to 48 wt.-% of a C12 fatty alcohol,
5 to 15 wt.-% of a C14 fatty alcohol,
4 to 13 wt.-% of a C16 fatty alcohol,
15 to 42 wt.-% of a C18 fatty alcohol, and
0 to 5 wt.-% of a C20 fatty alcohol,
each based on the total weight of the fatty alcohol composition. Said fatty alcohol composition is preferably obtained from oil extracted from Macaúba kernel.

In a preferred embodiment, the fatty alcohol composition comprises
3 to 7 wt.-%, preferably 4 to 6 wt.-%, of a C8 fatty alcohol,
2 to 6 wt.-%, preferably 3 to 5 wt.-%, of a C10 fatty alcohol,
36 to 46 wt.-%, preferably 38 to 42 wt.-%, of a C12 fatty alcohol,
6 to 13 wt.-%, preferably 8 to 11 wt.-%, of a C14 fatty alcohol,
5 to 11 wt.-%, preferably 6 to 9 wt.-%, of a C16 fatty alcohol,
25 to 40 wt.-%, preferably 30 to 38 wt.-% of a C18 fatty alcohol, and
0 to 4 wt.-%, preferably 0 to 3 wt.-%, of a C20 fatty alcohol,
each based on the total weight of the fatty alcohol composition. Said fatty alcohol composition is preferably obtained from oil extracted from Macaúba kernel.

In a preferred embodiment, the fatty alcohol composition comprises
0 to 5 wt.-%, preferably 0 to 3 wt.-%, and in particular 0 to 2 wt.-%, of a C10 fatty alcohol,
0 to 6 wt.-%, preferably 0 to 5 wt.-%, and in particular 1 to 4 wt.-%, of a C12 fatty alcohol,
0 to 6 wt.-%, preferably 0 to 5 wt.-%, and in particular 1 to 4 wt.-%, of a C14 fatty alcohol,
10 to 35 wt.-%, preferably 13 to 32 wt.-%, and in particular 15 to 30 wt.-%, of a C16 fatty alcohol,
55 to 85 wt.-%, preferably 60 to 80 wt.-%, and in particular 65 to 75 wt.-%, of a C18 fatty alcohol,
0 to 4 wt.-%, preferably 0 to 3 wt.-%, and in particular 0 to 2 wt.-%, of a C20 fatty alcohol,
each based on the total weight of the fatty alcohol composition. Said fatty alcohol composition is preferably obtained from oil extracted from Macaúba pulp.

In a preferred embodiment, the fatty alcohol composition comprises at least 45 wt.-% based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols or C12-C14 fatty alcohols.

In a preferred embodiment, the fatty alcohol composition comprises at least 85 wt.-% based on the total weight of the fatty alcohol composition, of C4-C22 fatty alcohols, preferably C10-C22 fatty alcohols, more preferably C12-C20 fatty alcohols, even more preferably C12-C20 fatty alcohols, and in particular C12-C18 fatty alcohols.

In a preferred embodiment, the fatty alcohol composition comprises at least 10 wt.-% of C16 fatty alcohols and at least 75 wt.-% of C18 fatty alcohols, each based on the total weight of the fatty alcohol composition.

In a preferred embodiment, the fatty alcohol composition comprises 10 to 25 wt.-% of C16 fatty alcohols and 75 to 90 wt.-% of C18 fatty alcohols, each based on the total weight of the fatty alcohol composition.

In a preferred embodiment, the fatty alcohol composition comprises at least 80 wt.-%, preferably at least 90 wt.-%, and in particular at least 95 wt.-%, based on the total weight of the fatty alcohol composition, of C12-14 fatty alcohols.

In a preferred embodiment, the fatty alcohol composition comprises at least 80 wt.-%, preferably at least 90 wt.-%, and in particular at least 95 wt.-%, based on the total weight of the fatty alcohol composition, of C12-18 fatty alcohols.

In a preferred embodiment, the fatty alcohol composition comprises at least 2 wt.-% of C10 fatty alcohols, at least 35 wt.-% of C12 fatty alcohols, at least 5 wt.-% of C14 fatty alcohols, and at least 4 wt.-% of C16 fatty alcohols, each based on the total weight of the fatty alcohol composition.

In a preferred embodiment, the fatty alcohol composition comprises 3 to 7 wt.-% of C8 fatty alcohols, 2 to 6 wt.-% of C10 fatty alcohols, 35 to 45 wt.-% of C12 fatty alcohols, 5 to 13 wt.-% of C14 fatty alcohols, and 4 to 10 wt.-% of C16 fatty alcohols, each based on the total weight of the fatty alcohol composition.

In a preferred embodiment, the fatty alcohol composition comprises at least 90 wt.-% of C8 fatty alcohols, based on the total weight of the fatty alcohol composition.

In a preferred embodiment, the fatty alcohol composition comprises at least 90 wt.-% of C10 fatty alcohols, based on the total weight of the fatty alcohol composition.

In a preferred embodiment, the fatty alcohol composition comprises at least 90 wt.-% of C12 fatty alcohols, based on the total weight of the fatty alcohol composition.

In a preferred embodiment, the fatty alcohol composition comprises at least 90 wt.-% of C14 fatty alcohols, based on the total weight of the fatty alcohol composition.

In a preferred embodiment, the fatty alcohol composition comprises at least 90 wt.-% of C16 fatty alcohols, based on the total weight of the fatty alcohol composition.

In a preferred embodiment, the fatty alcohol composition comprises at least 90 wt.-% of C18 fatty alcohols, based on the total weight of the fatty alcohol composition.

As indicated above, the present invention further relates to the use of a fatty alcohol composition as above-outlined for manufacturing a C4 fatty alcohol, a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, a C22 fatty alcohol, or blends thereof.

Preferably, the respective fatty alcohols are separated via fractionation of the alcohol composition or of its ester composition such as methylester composition. The skilled person is aware of suitable processes such as distillation. In a preferred embodiment, the alcohols are separated via fractional distillation. Preferably, the fractional distillation system is designed as add-on unit operations to produce high purity of single alcohol cuts. The single or two tower systems are preferably fitted with high performance structured packings for minimal pressure drop and highest product quality.

As indicated above, the present invention further relates to a process of manufacturing a surfactant comprising the steps of
A) separating fatty alcohols selected from the group consisting of a C6 fatty alcohol, a C8 fatty alcohol, a C10 fatty alcohol, a C12 fatty alcohol, a C14 fatty alcohol, a C16 fatty alcohol, a C18 fatty alcohol, and a C20 fatty alcohol, from the fatty alcohol composition as above-disclosed,
B) optionally blending at least two of the separated fatty alcohols,
C) subsequently converting at least one of the separated fatty alcohols selected from the group consisting of a C6 fatty alcohol, a C8 fatty alcohol, a C10 fatty alcohol, a C12 fatty alcohol, a C14 fatty alcohol, a C16 fatty alcohol, a C18 fatty alcohol, and a C20 fatty alcohol into the respective fatty alcohol derivative selected from the group consisting of fatty alcohol sulfate, fatty alcohol ethersulfate, fatty ester, fatty amine, fatty amide, fatty alcohol glycoside, fatty alkyl polyglucoside, fatty ether, and fatty alcohol ethoxylate.

It is to be understood that all preferred embodiment regarding the fatty alcohol composition as above-disclosed shall apply for the process of manufacturing a surfactant, as well. Thus, the fatty alcohol composition is derived from a plant or a fruit having an oil yield in tons per hectare per year of at least 6 t/ha/yr.

In a preferred embodiment, the fatty alcohol composition as above-disclosed applied in step A) is first blended with a fatty alcohol composition obtained from a plant having an oil yield in tons per hectare per year of less than 6 t/ha/yr, preferably of less than 5 t/ha/yr, more preferably of less than 4.5 t/ha/yr. In a preferred embodiment, the fatty alcohol composition as above-disclosed applied in step A) is first blended with a fatty alcohol composition obtained from a plant having an oil yield in tons per hectare per year of 0.1 to less than 6 t/ha/yr, preferably of 0.3 to 5 t/ha/yr, more preferably of 0.5 to 4.5 t/ha/yr. In a preferred embodiment, the fatty alcohol composition obtained from a plant having an oil yield in tons per hectare per year of less than 6 t/ha/yr is derived from coconut oil (CNO), palm oil (PO), and/or palm kernel oil (PKO). In another preferred embodiment the fatty alcohol composition obtained from a plant having an oil yield in tons per hectare per year of less than 6 t/ha/yr is derived from soy oil, sunflower oil, olive oil, and/or rapeseed oil.

In yet another preferred embodiment the fatty alcohol composition obtained from a plant having an oil yield in tons per hectare per year of less than 6 t/ha/yr is derived from coconut oil (CNO), palm oil (PO), palm kernel oil (PKO), soy oil, sunflower oil, olive oil, and/or rapeseed oil.

In a preferred embodiment, the fatty alcohol composition obtained from a plant having an oil yield in tons per hectare per year of less than 6 t/ha/yr is derived from coconut oil (CNO), and/or palm kernel oil (PKO).

In a preferred embodiment, the fatty alcohol composition obtained from a plant having an oil yield in tons per hectare per year of less than 6 t/ha/yr is derived from palm oil (PO) and/or palm kernel oil (PKO).

In a preferred embodiment, the fatty alcohol composition as above-disclosed applied in step A) obtained from a plant having an oil yield in tons per hectare per year of at least 6 t/ha/yr, which is derived from Macaúba kernel, is first blended with a fatty alcohol composition obtained from a plant having an oil yield in tons per hectare per year of less than 6 t/ha/yr, which is derived from palm kernel oil (PKO).

In a preferred embodiment, the fatty alcohol composition as above-disclosed applied in step A) obtained from a plant having an oil yield in tons per hectare per year of at least 6 t/ha/yr, which is derived from Macaúba kernel, is first blended with a fatty alcohol composition obtained from a plant having an oil yield in tons per hectare per year of less than 6 t/ha/yr, which is derived from coconut oil (CNO).

In a preferred embodiment, the fatty alcohol composition as above-disclosed applied in step A) obtained from a plant having an oil yield in tons per hectare per year of at least 6 t/ha/yr, which is derived from Macaúba kernel, is first blended with a fatty alcohol composition obtained from a plant having an oil yield in tons per hectare per year of less than 6 t/ha/yr, which is derived from palm kernel oil (PKO) and coconut oil (CNO).

In a preferred embodiment, the fatty alcohol composition as above-disclosed applied in step A) obtained from a plant having an oil yield in tons per hectare per year of at least 6 t/ha/yr, which is derived from Macaúba pulp, is first blended with a fatty alcohol composition obtained from a plant having an oil yield in tons per hectare per year of less than 6 t/ha/yr, which is derived from palm oil (PO).

In a preferred embodiment, the fatty alcohol derivative obtained in step C) is blended with fatty alcohol derivatives obtained from a fatty alcohol composition obtained from a plant having an oil yield in tons per hectare per year of less than 6 t/ha/yr, preferably of less than 5 t/ha/yr, more preferably of less than 4.5 t/ha/yr, and a subsequent conversion into the respective fatty alcohol derivatives. In this connection it is to be understood that the subsequent conversion preferably provide a fatty aclohol derivative, which is equal to the fatty alcohol derivative obtained in step C). Hence, if step C) provides a specific fatty amine, the additional fatty alcohol derivative blended into the mixture is also the specific fatty amine (however obtained from a plant having an oil yield in tons per hectare per year of less than 6 t/ha/yr).

In a preferred embodiment, the fatty alcohol composition obtained from a plant having an oil yield in tons per hectare per year of less than 6 t/ha/yr is derived from coconut oil (CNO), palm oil (PO), and/or palm kernel oil (PKO). In another preferred embodiment the fatty alcohol composition obtained from a plant having an oil yield in tons per hectare per year of less than 6 t/ha/yr is derived from soy oil, sunflower oil, olive oil, and/or rapeseed oil.

In yet another preferred embodiment the fatty alcohol composition obtained from a plant having an oil yield in tons per hectare per year of less than 6 t/ha/yr is derived from coconut oil (CNO), palm oil (PO), palm kernel oil (PKO), soy oil, sunflower oil, olive oil, and/or rapeseed oil.

In a preferred embodiment, the fatty alcohol composition obtained from a plant having an oil yield in tons per hectare per year of less than 6 t/ha/yr is derived from coconut oil (CNO), and/or palm kernel oil (PKO).

In a preferred embodiment, the fatty alcohol composition obtained from a plant having an oil yield in tons per hectare per year of less than 6 t/ha/yr is derived from palm oil (PO) and/or palm kernel oil (PKO).

In a preferred embodiment, the fatty alcohol derivative obtained in step C) obtained from a plant having an oil yield in tons per hectare per year of at least 6 t/ha/yr, which is derived from Macaúba kernel, is blended with fatty alcohol derivatives obtained from a fatty alcohol composition obtained from a plant having an oil yield in tons per hectare per year of less than 6 t/ha/yr, which is derived from palm kernel oil (PKO) and a subsequent conversion into the respective fatty alcohol derivatives.

In a preferred embodiment, the fatty alcohol derivative obtained in step C) obtained from a plant having an oil yield in tons per hectare per year of at least 6 t/ha/yr, which is derived from Macaúba kernel, is blended with fatty alcohol derivatives obtained from a fatty alcohol composition obtained from a plant having an oil yield in tons per hectare per year of less than 6 t/ha/yr, which is derived from coconut oil (CNO) and a subsequent conversion into the respective fatty alcohol derivatives.

In a preferred embodiment, the fatty alcohol derivative obtained in step C) obtained from a plant having an oil yield in tons per hectare per year of at least 6 t/ha/yr, which is derived from Macaúba kernel, is blended with fatty alcohol derivatives obtained from a fatty alcohol composition obtained from a plant having an oil yield in tons per hectare per year of less than 6 t/ha/yr, which is derived from palm kernel oil (PKO) and coconut oil (CNO) and a subsequent conversion into the respective fatty alcohol derivatives.

In a preferred embodiment, the fatty alcohol derivative obtained in step C) obtained from a plant having an oil yield in tons per hectare per year of at least 6 t/ha/yr, which is derived from Macaúba pulp, is blended with fatty alcohol derivatives obtained from a fatty alcohol composition obtained from a plant having an oil yield in tons per hectare per year of less than 6 t/ha/yr, which is derived from palm oil (PO) and a subsequent conversion into the respective fatty alcohol derivatives.

As indicated above, the present invention further relates to a surfactant obtained by the above-disclosed process. In this connection, suitable surfactants are selected from the group consisting of fatty alcohol sulfates, fatty alcohol ethersulfates, fatty esters, fatty amines, fatty amides, fatty alcohol glycosides, fatty alkyl polyglucoside, fatty ethers, and fatty alcohol ethoxylate.

Suitable fatty alcohol sulfates can be express by the general formula (I)

R-O-SO3M (I),

wherein R is saturated or unsaturated C4-C24-alkyl, preferably C6-C20-alkyl, and in particular C12-C14-alkyl, and M is a suitable counter ion, such as triethanolammonium, Na+, K+ or NH4+, preferably sodium.

Suitable fatty alcohol ethersulfates can be express by the general formula (II)

R-O(CH2CH2O)n-SO3M (II),

wherein R is saturated or unsaturated C4-C24-alkyl, preferably C6-C20-alkyl, and in particular C12-C14-alkyl, n is an average number of 1 to 30, preferably 1 to 10, more preferably of 1 to 5, and M is a suitable counter ion, such as triethanolammonium, Na+, K+ or NH4+, preferably sodium.

Suitable fatty esters can be expressed by the general formula (III)

R-O-CO-R1 (III),

wherein R is saturated or unsaturated C4-C24-alkyl, preferably C6-C20-alkyl, and in particular C12-C14-alkyl, and R1 is C1-C24-alkyl, C1-C4-alkoxy, aryl, benzyl, or heterocyclyl.

The term "fatty amines" as used herein encompasses primary fatty amines, ethoxylated fatty amines, fatty amine oxides, and amine salts.

Suitable fatty amines can be expressed by the general formula (IV)

R-NR2R2' (IV),

wherein R is saturated or unsaturated C4-C24-alkyl, preferably C6-C20-alkyl, and in particular C12-C14-alkyl, and R2 and R2' are independently H, C1-C4-alkyl, or C1-C8-alkylene-NR21R22, wherein R21 and R22 are independently C1-C4-alky.

Suitable ethoxylated fatty amines can be expressed by the general formula (V)

R-NR3R4 (V),

wherein R is saturated or unsaturated C4-C24-alkyl, preferably C6-C20-alkyl, and in particular C12-C14-alkyl, R3 and R4 are independently H or -(CH2CH2O)n-N, n is an average number of 1 to 20, preferably of 1.5 to 10, N is H or C1-C4-alkyl.

Suitable fatty amine oxides can be expressed by the general formula (VI)

R-NR5R6O (VI),

wherein R is saturated or unsaturated C4-C24-alkyl, preferably C6-C20-alkyl, and in particular C12-C14-alkyl, and R5 and R6 are independently C1-C4-alkyl.

Suitable amine salts are e.g. quaternary amines having the general formula (VII)

R-NR7R8R9Q (VII),

wherein R is saturated or unsaturated C4-C24-alkyl, preferably C6-C20-alkyl, and in particular C12-C14-alkyl, R7, R8, and R9 are independently H or C1-C16-alkyl, preferably H or C4-C10-alkyl, and Q is a halide, hydrogensulfate, bicarbonate, benzoate, formate, acetate, propionate and butyrate, preferably chloride. In this connection, it is to be understood that a positive charge is on the nitrogen and a negative charge is on Q.

In a preferred embodiment, the amine salt has the general formula (VII)

R-NR7R8R9Q (VII),

wherein R is saturated or unsaturated C12-C20-alkyl, preferably C14-C18-alkyl, and in particular C12-C14-alkyl, R7, R8, and R9 are independently H or C1-C5-alkyl, preferably methyl, and Q is a halide, preferably chloride.

Suitable fatty alcohol glycosides can be expressed by the general formula (VIII)

R-O-G (VIII),

wherein R is saturated or unsaturated C4-C24-alkyl, preferably C6-C20-alkyl, and in particular C12-C14-alkyl, and G is a glucose moiety, preferably connected via the C1 carbon atom.

Suitable fatty alkyl polyglucoside can be expressed by the general formula (IX)

R-O-(G)g-H (IX),

wherein R is saturated or unsaturated C4-C24-alkyl, preferably C6-C20-alkyl, and in particular C8-C10-alkyl or C8-C14-alkyl or C12-C14-alkyl, G is a glucose moiety, preferably connected via the C1 and C4 carbon atom, and g is an average number of 1 to 50, preferably of 5 to 20.

Suitable fatty ethers can be expressed by the general formula (X)

R-O-R10 (X),

wherein R is saturated or unsaturated C4-C24-alkyl, preferably C6-C20-alkyl, more preferably C8-C14-alkyl, and in particular C12-C14-alkyl, and R10 is C1-C4-alkyl, C1-C4-alkoxy, aryl, benzyl, or heterocyclyl.

Suitable fatty alcohol ethoxylate can be expressed by the general formula (XI)

R-O-(CH2CH2O)m-R11 (XI),

wherein R is saturated or unsaturated C4-C24-alkyl, preferably C6-C20-alkyl, more preferably C12-C18-alkyl, still more preferably C12-C16-alkyl, and in particular C12-C14-alkyl, m is an average number of 1 to 50, preferably of 1.5 to 10, and R11 is H, C1-C4-alkyl, C1-C4-alkoxy, aryl, benzyl, or heterocyclyl.

Suitable fatty amides can be expressed by the general formula (XII)

R-NR12-CO-R13 (XII),

wherein R is saturated or unsaturated C4-C24-alkyl, preferably C6-C20-alkyl, and in particular C12-C14-alkyl, and R12 is H or C1-C4-alkyl, preferably H, R13 is C3-C24-alkyl, C1-C4-alkoxy, aryl, benzyl, heterocyclyl, or C1-C8-alkylene-NR14R14', wherein R14 and R14' are independently C1-C4-alkyl.

As indicated above, the present invention relates to a process of manufacturing a C4 fatty alcohol, a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, a C22 fatty alcohol, and/or blends thereof, preferably a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, and/or blends thereof, more preferably a C8 fatty alcohol, a C10 fatty alcohol, a C12 fatty alcohol, a C14 fatty alcohol, a C16 fatty alcohol, a C18 fatty alcohol, and/or blends thereof, even more preferably a C10 fatty alcohol, a C12 fatty alcohol, a C14 fatty alcohol, a C16 fatty alcohol, and/or blends thereof, and in particular a C12 fatty alcohol, a C14 fatty alcohol, and/or blends thereof, comprising separating the C4 fatty alcohol, C6 fatty alcohol, C8 fatty alcohol, C10 fatty alcohol, C12 fatty alcohol, C14 fatty alcohol, C16 fatty alcohol, C18 fatty alcohol, C20 fatty alcohol, and/or C22 fatty alcohol, preferably the C6 fatty alcohol, C8 fatty alcohol, C10 fatty alcohol, C12 fatty alcohol, C14 fatty alcohol, C16 fatty alcohol, C18 fatty alcohol, and/or C20 fatty alcohol, more preferably the C8 fatty alcohol, C10 fatty alcohol, C12 fatty alcohol, C14 fatty alcohol, C16 fatty alcohol, and/or C18 fatty alcohol, even more preferably the C10 fatty alcohol, C12 fatty alcohol, C14 fatty alcohol, and/or C16 fatty alcohol, and in particular the C12 fatty alcohol and/or C14 fatty alcohol, from the fatty alcohol composition as above-disclosed and optionally blending at least two of the separated fatty alcohols.

It is to be understood that if the process provides a blend, that the blending step is not optional.

In a preferred embodiment, the present invention relates to a process of manufacturing a blend of a C10 fatty alcohol, a C12 fatty alcohol, a C14 fatty alcohol, and a C16 fatty alcohol, comprising separating the C10 fatty alcohol, the C12 fatty alcohol, the C14 fatty alcohol, and the C16 fatty alcohol from the fatty alcohol composition as above-disclosed and subsequently blending the separated C10 fatty alcohol, the separated C12 fatty alcohol, the separated C14 fatty alcohol, and the separated C16 fatty alcohol.

In a preferred embodiment, the present invention relates to a process of manufacturing a blend of a C12 fatty alcohol and a C14 fatty alcohol, comprising separating the C12 fatty alcohol and the C14 fatty alcohol from the fatty alcohol composition as above-disclosed and subsequently blending the separated C12 fatty alcohol and the separated C14 fatty alcohol.

The present invention also is directed to a process of manufacturing e.g. a C4 fatty alcohol, a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, and/or a C22 fatty alcohol, preferably a C6 fatty alcohol, a C8 fatty alcohol, a C10 fatty alcohol, a C12 fatty alcohol, a C14 fatty alcohol, a C16 fatty alcohol, a C18 fatty alcohol, and/or a C20 fatty alcohol, more preferably a C8 fatty alcohol, a C10 fatty alcohol, a C12 fatty alcohol, a C14 fatty alcohol, a C16 fatty alcohol, and/or a C18 fatty alcohol, even more preferably a C10 fatty alcohol, a C12 fatty alcohol, a C14 fatty alcohol, and/or a C16 fatty alcohol, and in particular a C12 fatty alcohol and/or a C14 fatty alcohol, comprising separating the C4 fatty alcohol, C6 fatty alcohol, C8 fatty alcohol, C10 fatty alcohol, C12 fatty alcohol, C14 fatty alcohol, C16 fatty alcohol, C18 fatty alcohol, C20 fatty alcohol, and/or C22 fatty alcohol, preferably the C6 fatty alcohol, C8 fatty alcohol, C10 fatty alcohol, C12 fatty alcohol, C14 fatty alcohol, C16 fatty alcohol, C18 fatty alcohol, and/or C20 fatty alcohol, more preferably the C8 fatty alcohol, C10 fatty alcohol, C12 fatty alcohol, C14 fatty alcohol, C16 fatty alcohol, and/or C18 fatty alcohol, even more preferably the C10 fatty alcohol, C12 fatty alcohol, C14 fatty alcohol, and/or C16 fatty alcohol, and in particular the C12 fatty alcohol and/or C14 fatty alcohol, from the fatty alcohol composition as above-disclosed.

As indicated above, the present invention further relates to the use of the above-outlined surfactant in a personal care composition, a cleaning composition, a nutrition formulation, a pharmaceutical formulation, or a crop formulation.

In a preferred embodiment, the above-outlined surfactants are used in a personal care composition, preferably selected from the group consisting of face care composition, hair care composition, body care composition, oral care composition, or antiperspirants and deodorants.

Suitable cosmetic formulations containing active ingredients are, e.g., hormone preparations, vitamin preparations, vegetable extract preparations and antibacterial preparations.

According to the present invention the personal care composition may comprise one or more active agent(s), e.g., organic and inorganic UV filters and vitamins, as well as other ingredients or additives, e.g., pigments, emulsifiers, emollients, viscosity regulators, stabilizers, preservatives, or fragrances.

In a preferred embodiment, the above-outlined surfactants are used in a sunscreen.

In a preferred embodiment, the above-outlined surfactants are used in a decorative preparations, preferably selected from the group consisting of lipsticks, nail varnishes, eye shadows, mascaras, dry and moist make-up, rouge, powders, depilatory agents and suntan lotions.

The personal care composition is preferably in form of creams, ointments, pastes, foams, gels, lotions, powders, make-ups, sprays, sticks or aerosols.

Preferably, the surfactant is used to control the metal ions, improve the dispersing, improve the emulsifying, control the foaming, modify the surface, and/or protect the active agent(s).

In a preferred embodiment, the above-outlined surfactants are used in a cleaning composition, preferably selected from the group consisting of home care formulation, industrial care formulation, and institutional care formulation.

In a preferred embodiment, the cleaning composition is selected from the group consisting of laundry composition (personal and commercial), dishwashing composition, hard surface cleaning composition, food service and kitchen hygiene composition, food and beverage processing composition, sanitation composition, institutional cleaning composition, industrial cleaning composition, and vehicle and transportation care composition.

The cleaning composition may comprise at least one bleaching system known in the art in an amount of from 0 to 50 wt.-%. Suitable bleaching components include bleaching catalysts, photobleaches, bleach activators, sources of hydrogen peroxide such as sodium percarbonate and sodium perborates, preformed peracids, and mixtures thereof.

The cleaning compositions may furthermore comprise dirt-suspending agents, for example sodium carboxymethylcellulose; pH regulators, for example alkali metal or alkaline earth metal silicates; bactericides; foam regulators, for example soap; salts for adjusting the spray drying and the granulating properties, for example sodium sulfate; fragrances; antistatic agents; fabric conditioners; further bleaching agents; pigments; and/or toning agents.

Preferably, the surfactant is used to control the metal ions, improve the dispersing, improve the emulsifying, control the foaming, modify the surface, and/or protect the ingredient(s).

In a preferred embodiment, the above-outlined surfactants are used in a nutrition formulation, preferably from the group selected from food formulations and feed formulations. The nutrition formulation can have any suitable form, e.g. liquid or solid and can be administered or uptaken in any suitable manner, e.g. orally, parenterally, or rectally.

For the preparation of a nutrition formulation, or a premix or a precursor, the process may comprise mixing a stabilized solid and/or liquid formulation comprising at least one or more food substance(s) and at least one additional ingredient(s) such as stabilizing agent.

Suitable stabilizing agents may be selected from the group consisting of gummi arabicum, at least one plant protein and mixtures thereof. It is understood that the stabilizing agent can be selected from one agent, e.g. only gummi arabicum or be composed of a mixture of e.g. one plant protein and gummi arabicum or a mixture of two or three or more different plant proteins. In one embodiment, the stabilizing agent is gummi arabicum. In another embodiment, the stabilizing agent is at least one plant protein.

Preferably, the surfactant is used to control the metal ions, improve the dispersing, improve the emulsifying, control the foaming, modify the surface, and/or protect the ingredient(s).

In a preferred embodiment, the above-outlined surfactants are used in pharmaceutical formulation. The pharmaceutical formulation may be administered in any suitable manner such as by oral, transdermal, parenteral, nasal, vaginal, or rectal application. Suitable solid pharmaceutical formulation can be in form of tablets, suppositories, or capsules or in form of a spray. Suitable transdermal pharmaceutical formulations encompass patches or formulations such as sprays, lotions, creams, oils, foams, ointments, powders, or gels. Suitable liquid pharmaceutical formulations are preferably administered orally, parenterally, or nasal.

The pharmaceutical formulation is preferably in solid, semi-solid, or liquid form, preferably in form of tablets, suppositories, capsules, patches, as sprays, lotions, creams, oils, foams, ointments, powders, gels, or fluid.

The pharmaceutical formulation comprises at least one active agent, e.g. selected from the group consisting of anti-cancer agent, hormone, antiviral agent, antifungal agent, antibacterial agent, and inhibitor.

Preferably, the surfactant is used to control the metal ions, improve the dispersing, improve the emulsifying, control the foaming, modify the surface, and/or protect the active agent(s).

In a preferred embodiment, the above-outlined surfactants are used in crop formulation, preferably selected from the group consisting of pesticide formulations, fungicide formulations, and herbicide formulations.

The crop formulation is preferably in solid, semi-solid, or liquid form. Preferably, the crop formulation is suitable for a ready to use spray.

In a preferred embodiment, the pesticide formulation comprises a pesticide selected from the group consisting of chlorpyrifos, endosulfan, imazalil, DDT, toxaphene, lindane, methoxychlor, dieldrin, kelthane, chlordane, Perthane, endrin, aldrin, and heptachlor.

In a preferred embodiment, the fungicide formulation comprises a fungicide selected from the group consisting of azoxystrobin, pyraclostrobin, fluoxastrobin, trifloxystrobin, picoxystrobin, epoxiconazole, prothioconazole, myclobutanil, tebuconazole, propiconazole, cyproconazole, fenbuconazole, boscalid, penthiopyrad, bixafen, isopyrazam, sedaxane, fluopyram, and thifluzamide.

In a preferred embodiment, the herbicide formulation comprises a herbicide selected from the group consisting of glyphosate, glufosinate, imidazolinone (such as imazamethabenz, imazamox, imazapic (e.g. Kifix), imazapyr, imazaquin and imazethapyr), and cyclohexanediones (such as tepraloxydim and clethodim).

Suitable herbicide formulation show enhanced herbicide action against undesirable harmful plants, in particular against *Acalypha* species such as *Acalypha indica*, *Dinebra* species such as *Dinebra Arabica, Cynotis spec such as Cynotis axillaris*, *Parthenium spec such as Parthenium hysterophorus, Physalis spec such as Physalis minima, Digera spec such as Digera arvensis, Alopecurus myosuroides*, *Apera spicaventi*, *Brachiaria* spec. such as *Brachiaria deflexa* or *Brachiaria plantaginea*, *Echinochloa* spec. such as *Echinochloa colonum, Leptochloa* spec. such as *Leptochloa fusca*, Rottboellia cochinchinensis, *Digitaria sanguinalis, Eleusine indica,* Saccharum spontaneum, Cynodon dactylon, *Euphorbia hirta, Euphorbia geniculata, Commelina benghaiensis, Commelina communis, certain undesired Oryza spec. such as weedy rice or red rice (Oryza sativa), Phaiaris spec. such as Phalaris canariensis,* Celosia argentea, Xanthium strumarium, *Papaver rhoeas*, *Geranium* spec, *Brassica* spec, *Avena fatua*, *Bromus* spec., *Lolium* spec., *Phalaris* spec., *Setaria* spec., *Digitaria* spec., *brachiaria* spec., *Amaranthus* spec., *Chenopodium* spec., *Abutilon theophrasti*, *Galium aparine, Veronica* spec., or *Solanum* spec. and/or to improve their compatibility with crop plants, such as soybean, peanut, pea, bean, lentil, green gram, black gram, cluster bean, fenugreek, palm, other pulse or leguminous crops, or crops which are tolerant to the action of acetohydroxyacid synthase inhibiting herbicides, such as for example Clearfield^{®} wheat, Clearfield^{®} barley, Clearfield^{®} corn, Clearfield^{®} lentil, Clearfield^{®} oilseed rape or canola, Clearfield^{®} rice, Cultivance^{®} soybean and/or Clearfield^{®} sunflower. The formulation should also have a good pre-emergence herbicidal activity.

Preferably, the surfactant is used to control the metal ions, improve the dispersing, improve the emulsifying, control the foaming, modify the surface, and/or protect the crop.

In a preferred embodiment, the personal care composition, the cleaning composition, the nutrition formulation, the pharmaceutical formulation, or the crop formulation comprises at least two surfactants. In this connection it is to be understood that the personal care composition, the cleaning composition, the nutrition formulation, the pharmaceutical formulation, or the crop formulation may comprise at least two above-outlined surfactant, at least three of the above-outlined surfactant or at least one of the above-outlined surfactant in combination with at least one further, different surfactant. The at least one further, different surfactant may be fatty acid-based surfactants (e.g. sulfonates, amides, isethionates, taurates, glycolipids, amino acids, esterquats, sophorolipids, rhamnolipids, amphoacetates, betains, amido alkanolamides, alkoxylated fatty acid ester and fatty acid methyl esters or its sulfonates).

Potential mixtures of one of the above-outlined surfactants are listed in the following.

| | | | |
|---|---|---|---|
| 1 | + fatty acid methyl ester or its sulfonate | 12 | + amphotenside |
| 2 | + alkanolamide | 13 | + sulfoacetate |
| 3 | + isethionate | 14 | + alkylbetaine |
| 4 | + N-acyl-aminoacid (e.g. N-acylglutamic acid) | 15 | + alkylethoxylate |
| 5 | + taurate | 16 | + cationic polymer |
| 6 | + aminooxide | 17 | + cationic surfactant |
| 7 | + sulfonate | 18 | + silicone |
| 8 | + carboxylate | 19 | + sulfonated fatty acid salts |
| 9 | + sulfosuccinate | 20 | + proteinhydrolysate |
| 10 | + (alkylether)sulfate | 21 | + protein-derivative |
| 11 | + betaine (e.g. cocamindopropylbetaine) | 22 | + fatty alkyl polyglucoside |

As indicated above, the present invention further relates to a personal care composition, a cleaning composition, a nutrition formulation, a pharmaceutical formulation, or a crop formulation comprising a surfactant as above-outlined in more detail. It is to be understood that the further specification of the use of the surfactants in the respective personal care composition, a cleaning composition, a nutrition formulation, a pharmaceutical formulation, or a crop formulation also applies for the personal care composition, a cleaning composition, a nutrition formulation, a pharmaceutical formulation, or a crop formulation.

### Examples

The present invention is further illustrated by the following prophetic examples.

The following examples are considered for the Macaúba palm (e.g. having registration number AEB402A) having an oil yield in tons per hectare per year of about 9.0 t/ha/yr.

### Example 1

The Macaúba palm is planted on a cattle field, e.g. about 380 trees per hectare. No deforestation is needed since the Macaúba palms are cultivated on already existing fields (silvopastoral) and the farmer can in addition to cattle breeding and/or milk production distribute the Macaúba fruits.

### Example 2

The Macaúba palm is planted on soya plantation (having a growth height of about 20 to 80 cm and an oil yield in tons per hectare per year about 0.6 t/ha/yr), e.g. about 340 trees per hectare. Again, no deforestation is needed since the Macaúba palms are cultivated on an already existing plantation (agroforestry). As the Macaúba palm grows up to about 15 meters in height, the soya can be cultivated parallel. In this connection, it is also possible to cultivate at least one more additional different plant (having a growth height of about 1 to 7 m) such as sunflower (having an oil yield in tons per hectare per year of about 0.7 t/ha/yr) or beans parallel.

As can be seen from the above examples, deforestation can be significantly reduced by cultivating Macaúba palms. Further, the biodiversity can be increased. In addition, even if the Macaúba palm is not cultivated as a monoculture, the total oil yield can be comparable with an oil palm (having an oil yield in tons per hectare per year of about 3.8 t/ha/yr) monoculture since the oil yield as above-defined of the Macaúba palm is higher. Without being bound to any theory, using a plant having an improved oil yield, degraded areas and springs and watersheds can more easily recover. Further, the retention of moisture in the soil is improved.

## Claims

1. A process of manufacturing a fatty alcohol composition, the process comprising the step of
a) converting oil extracted from a plant having an oil yield in tons per hectare per year of at least 6 t/ha/yr into the fatty alcohol composition.

2. The process according to claim 1, wherein the plant is a palm, preferably a Macaúba palm, and in particular *Acrocomia aculeata* and/or
wherein the plant is a palm and the oil is extracted from the palm pulp and/or the palm kernel, preferably wherein the plant is Macaúba palm and the oil is extracted from the Macaúba kernel, and in particular wherein the plant is *Acrocomia aculeata* and the oil is extracted from *Acrocomia aculeata* kernel.

3. The process according to claim 1 or 2, wherein in step a) the conversion is conducted under chemical or enzymatic conditions, preferably under chemical conditions.

4. The process according to any one of claims 1 to 3, wherein step a) involves
i) a hydrogenation, preferably a transesterification and a hydrogenation, in particular a transesterification followed by a hydrogenation or
ii) a hydrolysis, preferably a hydrolysis and a hydrogenation, in particular a hydrolysis followed by a hydrogenation.

5. The process according to any one of claims 1 to 4, wherein the fatty alcohol composition comprises at least 45 wt.-%, based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols.

6. The process according to any one of claims 1 to 5, wherein the plant has an oil yield in tons per hectare per year in the range of 6 to 30 t/ha/yr, preferably 7 to 20 t/ha/yr, more preferably of 8 to 15 t/ha/yr.

7. The process according to any one of claims 1 to 6, wherein the process further comprises b) conversion of the fatty alcohols into derivatives thereof selected from the group consisting of fatty alcohol sulfates, fatty alcohol ethersulfates, fatty esters, fatty amines, fatty amides, fatty alcohol glycosides, fatty alkyl polyglucoside, fatty ethers, and fatty alcohol ethoxylate.

8. A fatty alcohol composition obtained by a process according to any one of claims 1 to 7.

9. A fatty alcohol composition obtained from the fruits of a plant having an oil yield in tons per hectare per year of at least 6 t/ha/yr, wherein the oil obtained from the plant is converted into the fatty alcohol composition.

10. The fatty alcohol composition according to claim 10, wherein the plant is a palm, preferably a Macaúba palm, and in particular *Acrocomia aculeata* and/or wherein the oil is obtained by extraction of the fruits, preferably wherein the plant is a palm and the oil is extracted from the palm pulp and/or the palm kernel, more preferably wherein the plant is Macaúba palm and the oil is extracted from the Macaúba kernel, and in particular wherein the plant is *Acrocomia aculeata* and the oil is extracted from *Acrocomia aculeata* kernel.

11. The fatty alcohol composition according to any one of claims 8 to 10, comprising at least 45 wt.-%, based on the total weight of the fatty alcohol composition, of C4-C22 fatty alcohols, preferably C6-C20 fatty alcohols, more preferably C8-C18 fatty alcohols, even more preferably C8-C16 fatty alcohols or C16-C18 fatty alcohols, and in particular C10-C16 fatty alcohols or C12-C14 fatty alcohols.

12. Use of a fatty alcohol composition according to any one of claims 8 to 11 for manufacturing a C4 fatty alcohol, a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, a C22 fatty alcohol, or blends thereof.

13. A process of manufacturing a surfactant comprising the steps of
A) separating fatty alcohols selected from the group consisting of a C6 fatty alcohol, a C8 fatty alcohol, a C10 fatty alcohol, a C12 fatty alcohol, a C14 fatty alcohol, a C16 fatty alcohol, a C18 fatty alcohol, and a C20 fatty alcohol, from the fatty alcohol composition according to any one of claims 8 to 11,
B) optionally blending at least two of the separated fatty alcohols,
C) subsequently converting at least one of the separated fatty alcohols selected from the group consisting of a C6 fatty alcohol, a C8 fatty alcohol, a C10 fatty alcohol, a C12 fatty alcohol, a C14 fatty alcohol, a C16 fatty alcohol, a C18 fatty alcohol, and a C20 fatty alcohol into the respective fatty alcohol derivative selected from the group consisting of fatty alcohol sulfate, fatty alcohol ethersulfate, fatty ester, fatty amine, fatty alcohol glycoside, fatty alkyl polyglucoside, fatty ether, and fatty alcohol ethoxylate.

14. The process according to claim 13, wherein the fatty alcohol composition according to any one of claims 8 to 11 applied in step A) is first blended with a fatty alcohol composition obtained from a plant having an oil yield in tons per hectare per year of less than 6 t/ha/yr, preferably wherein the fatty alcohol composition obtained from a plant having an oil yield in tons per hectare per year of less than 6 t/ha/yr is derived from coconut oil (CNO), palm oil (PO), and/or palm kernel oil (PKO) or
wherein the fatty alcohol derivative obtained in step C) is blended with fatty alcohol derivatives obtained from a fatty alcohol composition obtained from a plant having an oil yield in tons per hectare per year of less than 6 t/ha/yr and a subsequent conversion into the respective fatty alcohol derivatives, preferably wherein the fatty alcohol composition obtained from a plant having an oil yield in tons per hectare per year of less than 6 t/ha/yr is derived from coconut oil (CNO), palm oil (PO), and/or palm kernel oil (PKO).

15. A surfactant obtained by the process according to claim 13 or 14.

16. A process of manufacturing a C4 fatty alcohol, a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, a C22 fatty alcohol, and/or blends thereof, preferably a C6 fatty alcohol, a C8 fatty alcohol, a C10 fatty alcohol, a C12 fatty alcohol, a C14 fatty alcohol, a C16 fatty alcohol, a C18 fatty alcohol, a C20 fatty alcohol, and/or blends thereof, more preferably a C8 fatty alcohol, a C10 fatty alcohol, a C12 fatty alcohol, a C14 fatty alcohol, a C16 fatty alcohol, a C18 fatty alcohol, and/or blends thereof, even more preferably a C10 fatty alcohol, a C12 fatty alcohol, a C14 fatty alcohol, a C16 fatty alcohol, and/or blends thereof, and in particular a C12 fatty alcohol, a C14 fatty alcohol, and/or blends thereof, comprising separating the C4 fatty alcohol, C6 fatty alcohol, C8 fatty alcohol, C10 fatty alcohol, C12 fatty alcohol, C14 fatty alcohol, C16 fatty alcohol, C18 fatty alcohol, C20 fatty alcohol, and/or C22 fatty alcohol, preferably the C6 fatty alcohol, C8 fatty alcohol, C10 fatty alcohol, C12 fatty alcohol, C14 fatty alcohol, C16 fatty alcohol, C18 fatty alcohol, and/or C20 fatty alcohol, more preferably the C8 fatty alcohol, C10 fatty alcohol, C12 fatty alcohol, C14 fatty alcohol, C16 fatty alcohol, and/or C18 fatty alcohol, even more preferably the C10 fatty alcohol, C12 fatty alcohol, C14 fatty alcohol, and/or C16 fatty alcohol, and in particular the C12 fatty alcohol and/or C14 fatty alcohol, from the fatty alcohol composition according to any one of claims 8 to 11 and optionally blending at least two of the separated fatty alcohols.

17. Use of the surfactant according to claim 15 in a personal care composition, a cleaning composition, a nutrition formulation, a pharmaceutical formulation, or a crop formulation.

18. A personal care composition, a cleaning composition, a nutrition formulation, a pharmaceutical formulation, or a crop formulation comprising a surfactant according to claim 15.
